(19) Europäisches Patentamt
European Patent Office
Office européen des brevets

(11) Veröffentlichungsnummer: 0 224 789 B1

# (12) EUROPÄISCHE PATENTSCHRIFT

(45) Veröffentlichungstag der Patentschrift: **09.08.89**

(51) Int. Cl.⁴: **C07D 231/06**, D06L 3/12

(21) Anmeldenummer: **86115952.3**

(22) Anmeldetag: **18.11.86**

## (54) Pyrazolinverbindungen.

(30) Priorität: **29.11.85 DE 3542235**
**21.12.85 DE 3545604**

(43) Veröffentlichungstag der Anmeldung:
**10.06.87 Patentblatt 87/24**

(45) Bekanntmachung des Hinweises auf die Patenterteilung:
**09.08.89 Patentblatt 89/32**

(84) Benannte Vertragsstaaten:
CH DE FR GB IT LI

(56) Entgegenhaltungen:
**EP-A- 0 073 996**
**EP-A- 0 156 197**
**DE-A- 2 700 996**
**DE-A- 2 755 023**
**US-A- 3 131 079**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk(DE)**

(72) Erfinder: **Seng, Florin, Am Katterbach 46,**
**D-5060 Bergisch Gladbach 2(DE)**
Erfinder: **Schellhammer, Carl-Wolfgang, Dr.,**
**Katharinental 26, D-5060 Bergisch Gladbach 2(DE)**

EP 0 224 789 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand sind Diarylpyrazolin-Verbindungen, die eine über einen Alkylsulfonylrest gebundene externe Aminogruppe aufweisen.

Es ist allgemein bekannt, daß Weißtöner dieser Verbindungsklasse eine geringe Lagerstabilität ihrer wäßrigen Lösungen aufweisen, wenn jener Alkylenrest eine Ethylenbrücke ist.

Deshalb ist bereits vorgeschlagen worden, dieses Brückenglied durch längerkettige oder speziell verzweigte Alkylengruppen zu ersetzen (vgl. DE-A 2 700 996 und 2 755 023 sowie EP-A 156 197).

Diese Modifizierung der aus US 3 131 079 grundsätzlich bekannten Verbindungsklasse hat jedoch häufig zu einer Verringerung der Weißkraft oder der Brillianz sowie zu einer Verschiebung der Nuance zu weniger erwünschten Farbtönen geführt – ganz abgesehen davon, daß nicht immer die angestrebte hohe Lagerbeständigkeit erreicht worden ist.

Es wurde nun überraschenderweise gefunden, daß man Pyrazoline mit verbesserter Aufhellwirkung und einer angenehmen Rotnuance erhält, wenn man als Brückenglied einen Rest der Formel

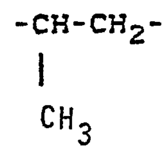

einführt.

Die neuen Pyrazolinverbindungen entsprechen der Formel

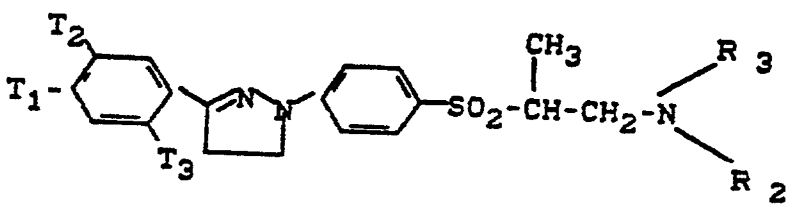 (I)

worin

$R_2$ und $R_3$ Methyl oder Ethyl,
$T_1$ und $T_2$ H oder Cl und
$T_3$ H oder $CH_3$ bedeuten,
sowie deren Quaternierungs- und Protonierungsprodukte.

Die neuen Pyrazolinverbindungen der Formel I können beispielsweise dadurch hergestellt werden, daß man in an sich bekannter Weise Ketonverbindungen der Formel

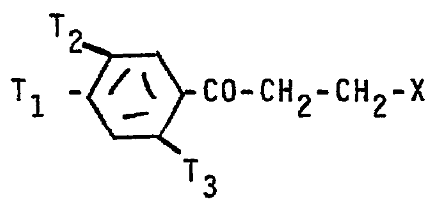 (II)

worin X eine Abgangsgruppe ist, mit Hydrazinverbindungen der Formel

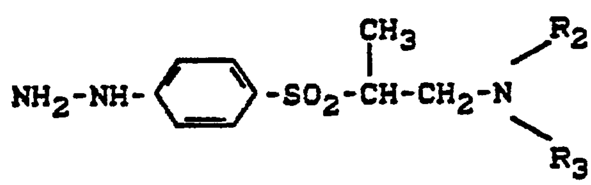 (III)

umsetzt und das Reaktionsprodukt gewünschtenfalls quaterniert oder protoniert.

Geeignete Quaternierungs- und Protonierungsprodukte sind solche, die man aus den "neutralen" Verbindungen der Formel I durch Behandlung mit in der Farbstoff- oder Aufheller-Chemie üblichen Quaternierungs- und Protonierungsmitteln, wie sie z.B. in der DE-OS 2 821 116 oder US-PS 4 051 117 beschrieben sind, erhält.

Bevorzugte Mittel dieser Art sind Dimethylsulfat, Diethylsulfat, Chlorwasserstoffsäure, gegebenenfalls durch Cl oder $CH_3$ substituierte Benzolsulfonsäure, Ethylenoxid, Propylenoxid, Ameisensäure, Essigsäure und Milchsäure.

Geeignete Abgangsgruppen X sind Halogenatome (vorzugsweise Cl) und Di–$C_1$–$C_4$–alkylaminogruppen.

Die Hydrazinverbindungen der Formel III erhält man nach folgenden Reaktionsschema:

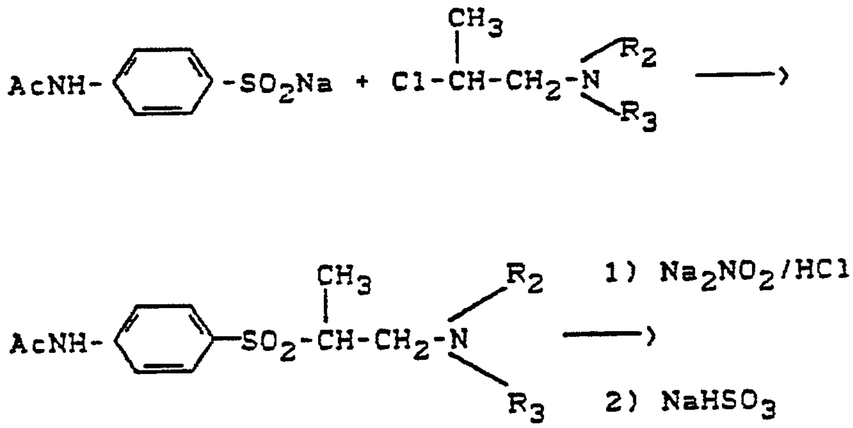


(III)

(Ac = Acylrest z.B. Acetyl)

Die Kondensation des Sulfinats mit dem Chloramin erfolgt bei erhöhten Temperaturen (100–200°C, in einem indifferenten Lösungsmittel, z.B. in siedendem Glykolmonomethylether.

Die anschließende Hydrazinsynthese wird unter üblichen Bedingungen durchgeführt.

Die neuen Pyrazolinverbindungen zeigen in gelöstem oder feinverteiltem Zustand eine starke Fluoreszenz und eignen sich hervorragend als optische Aufheller. Während die "neutralen" Verbindungen insbesondere zum Weißtönen von Textilmaterialien aus hydrophoben Fasern, insbesondere Polyamidfasern eingesetzt werden, dienen die bevorzugten quaternierten und protonierten Produkte zum Aufhellen von sauer-modifizierten Fasermaterialien, wie z.B. Acrylfasern, nach üblichen Färbemethoden und zum Aufhellen von Spinnmassen.

Diese bevorzugten Weißtöner entsprechen der Formel

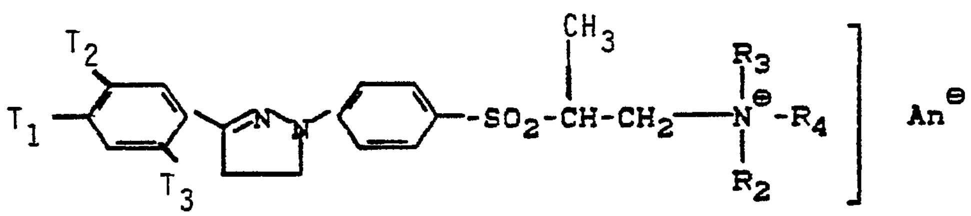

(V)

worin

$R_2$, $R_3$, $T_1$, $T_2$, $T_3$ die bei der Definition für Formel I angegebene Bedeutung haben,

$R_4$ Wasserstoff, $C_1$–$C_4$–Alkyl oder $C_2$–$C_4$–Hydroxyalkyl und

$An^{\ominus}$ ein farbloses Anion, das sich von den obengenannten Quaternierungs- und Protonierungsmitteln ableitet, bedeutet.

Die folgenden Beispiele dienen der näheren Erläuterung der vorliegenden Erfindung.

Beispiel 1

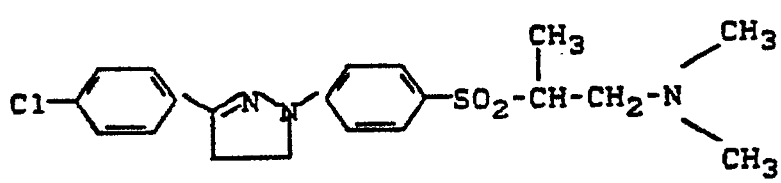

20,3 g (0,1 Mol) 3-Chlor-1-(4-chlorphenyl)-propanon-(1) werden zusammen mit 25,7 g (0,1 Mol) 4-(3-Dimethyl-amino-1-methyl-ethylsulfonyl)-phenylhydrazin in 150 ml Glykolmonomethylether und 12,5 ml Essigsäure 15 Std. bei 115° gerührt. Anschließend wird das Lösungsmittel bei einer Badtemperatur von 50°C im Wasserstrahlvakuum abdestilliert und der Rückstand in 100 ml Wasser aufgenommen. Die klare Lösung neutralisiert man mit Natriumbicarbonat. Dabei scheiden sich 31,2 g (77% d.Th.) 1-[4-(2-Dimethylamino-1-methyl-ethyl-sulfonylphenyl)]-3-(4-chlorphenyl)-pyrazolin (2) als gelbe Kristalle ab, die

nach dem Umlösen aus Ethanol bei 150–2°C schmelzen $\lambda_{max}$ = 369 nm (in DMF).

Im Vergleich zu der entsprechenden Verbindung mit einer geradkettigen Propylenbrücke gemäß DE-A 2 755 023 weist diese Verbindung eine deutlich verbesserte Weißkraft auf.

Gegenüber vergleichbaren Verbindungen gemäß DE-A 2 700 966 zeichnet sich die obengenannte Verbindung durch eine leicht erhöhte Brillianz und eine etwas rötere Nuance aus.

Beispiel 2 bis 4

Analog zu Beispiel 1 werden folgende Verbindungen hergestellt:

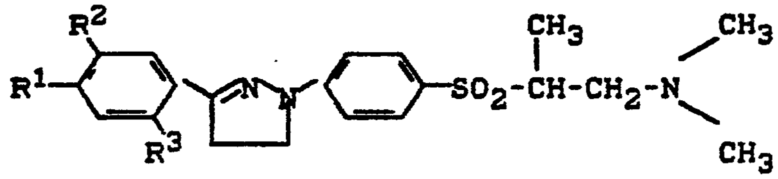

| | $R^1$ | $R^2$ | $R^3$ | $\lambda$-max (DMF) |
|---|---|---|---|---|
| 2) | H | H | H | 363 nm |
| 3) | Cl | Cl | H | 375,1 nm |
| 4) | Cl | Cl | $CH_3$ | 369 nm |

Beispiel 5

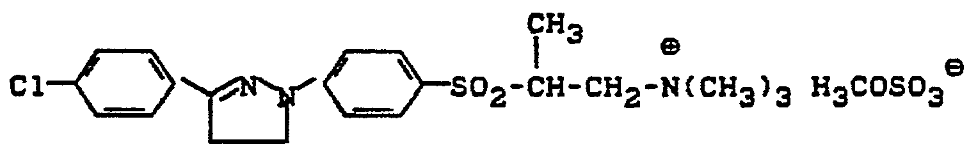

4,0 g (0,01 Mol) 1-[4-(2-Dimethylamino-1-methyl-ethyl-sulfonyl-phenyl)]-3-(4-chlorphenyl)-pyrazolin (2) werden in 70 ml Chlorbenzol gelöst und mit 2 g Dimethylsulfat versetzt. Die Lösung wird sofort trüb. Man rührt nach 20 Std. bei Raumtemperatur und saugt anschließend ab. Man erhält 4,7 g (88,4% d.Th.) 1-[4-(2-Trimethylammonium-1-methyl-ethyl-sulfonyl-phenyl)]-3-(4-chlorphenyl)-pyrazolin-(2)-methosulfat in Form gelber Kristalle, die nach dem Umlösen aus Methanol bei 135°C unter Zersetzung schmelzen.

$\lambda_{max}$ = 366,8 nm (in $H_2O$)

Analog wurden folgende Pyrazoline hergestellt:

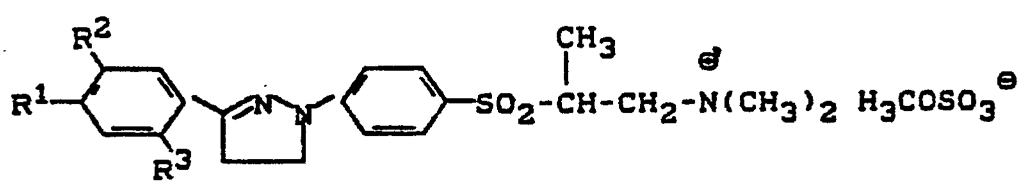

| | $R^1$ | $R^2$ | $R^3$ | max ($H_2O$) |
|---|---|---|---|---|
| 6) | H | H | H | 363.4 nm |
| 7) | Cl | Cl | H | 376 nm |
| 8) | Cl | Cl | $CH_3$ | 368,7 nm |

Beispiel 9

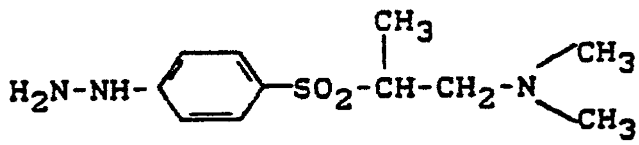

142 g (0,5 Mol) 4-(2-Dimethylamino-1-methyl-ethylsulfonyl)-acetanilid werden in einer Mischung aus 750 ml 37%iger Salzsäure und 750 ml Wasser 30 min bei 100°C gerührt. Anschließend wird bei 0 bis 5°C mit 35 g Natriumnitrit, gelöst in 990 ml Wasser diazotiert. Die erhaltene Diazoniumsalzlösung läßt man innerhalb einer Stunde bei 0 bis 8°C in eine Mischung aus 375 ml Wasser und 375 ml 40%iger Natriumbisulfitlösung fließen, wobei man durch Zugabe von insgesamt 78 g 45%iger Natronlauge den pH-Wert bei 7 hält. Anschließend rührt man eine Stunde bei Raumtemperatur, gibt dann 175 g 37%ige Salzsäure zu und rührt eine weitere Stunde bei 100°C. Zur Entfernung des Schwefeldioxides leitet man während des Abkühlens 4 Stunden lang gasförmigen Stickstoff durch den Ansatz. Danach wird mit 300 g 45%iger Natronlauge alkalisch gestellt und das dabei abgeschiedene 4-(2-Dimethylamino-1-methyl-ethylsulfonyl)-phenylhydrazin in 200 ml Diethylether aufgenommen. Nach dem Trocknen über Natriumsulfat und Abdestillation des Ethers hinterbleiben 98 g Hydrazin als gelbbraunes Öl.

Beispiel 10

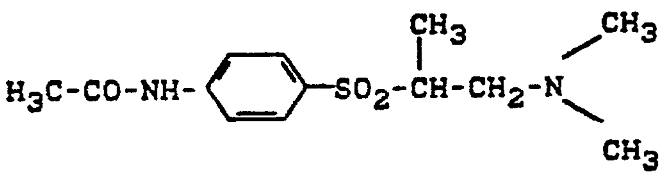

Eine Mischung aus 45,6 g (0,23 Mol) 4-Acetyl-amino-benzolsulfinsäure, 22 g Natriumbicarbonat und 28 g (0,23 Mol) 1-Dimethylamino-2-chlorpropan in 180 ml Glykol-monomethylether und 25 ml Wasser wird 6 Stunden bei 115°C gerührt. Anschließend wird das Lösungsmittel im Wasserstrahlvakuum abdestilliert und der Rückstand in 200 ml Wasser gelöst. Beim Versetzen mit 25 g 45%iger Natronlauge scheiden sich 53 g 4-(2-Dimethylamino-1-methyl-ethyl-sulfonyl)-acetanilid als weiße Kristalle ab, die bei 87–9°C schmelzen.

**Patentansprüche**

1. Pyrazolinverbindungen der Formel

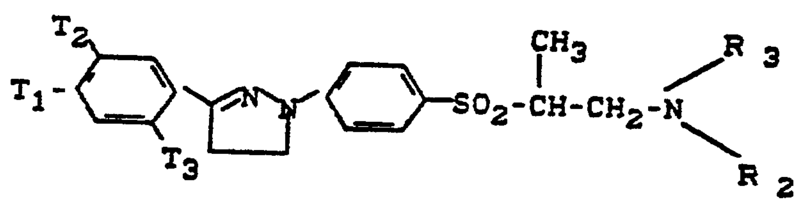

(I)

worin

$R_2$ und $R_3$ Methyl oder Ethyl,
$T_1$ und $T_2$ H oder Cl und
$T_3$ H oder $CH_3$ bedeuten,
sowie deren Quaternierungs- und Protonierungsprodukte.

2. Pyrazolinverbindung gemäß Anspruch 1 der Formel

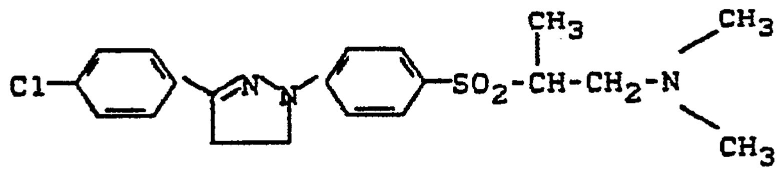

sowie deren Quaternierungs- und Protonierungsprodukte.

3. Verwendung der Pyrazolinverbindungen gemäß Anspruch 1 u. 2 als optische Aufheller.

**Claims**

1. Pyrazoline compounds of the formula

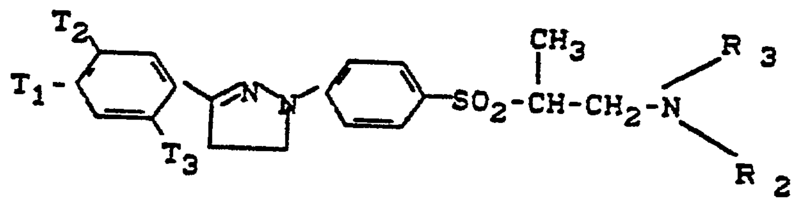

(I)

wherein

$R_2$ and $R_3$ denote methyl or ethyl,
$T_1$ and $T_2$ denote H or Cl and
$T_3$ denotes H or $CH_3$,
and also quaternization and protonation products thereof.

2. Pyrazoline compound according to Claim 1, of the formula

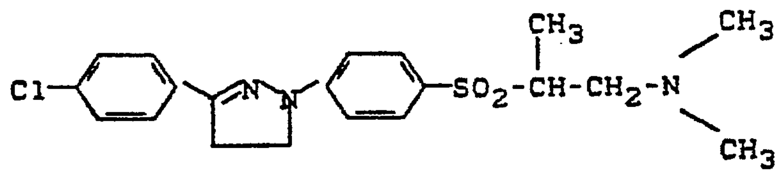

and also quaternization and protonation products thereof.

3. The use of the pyrazoline compounds according to Claim 1 and 2 as optical brighteners.

**Revendications**

1. Dérivés de la pyrazoline répondant à la formule

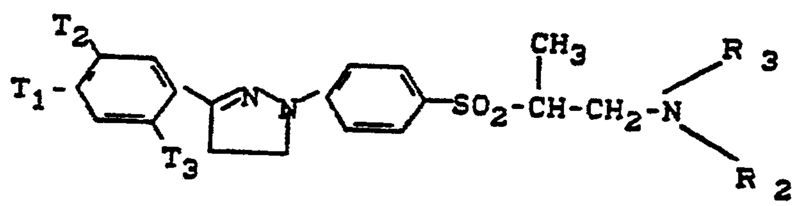

(I)

dans laquelle

$R_2$ et $R_3$ représentent des groupes méthyle ou éthyle,
$T_1$ et $T_2$ représentent H ou Cl, et
$T_3$ représente H ou $CH_3$,
et leurs produits de quaternisation et de protonisation.

2. Dérivé de la pyrazoline selon la revendication 1, répondant à la formule

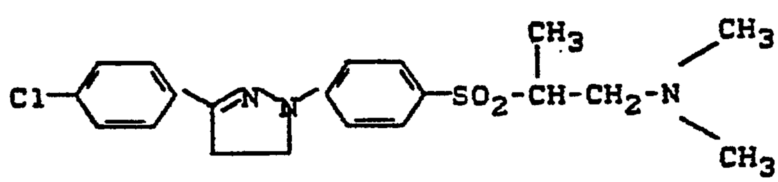

et ses produits de quaternisation et de protonisation.

3. Utilisation des dérivés de la pyrazoline selon les revendications 1 et 2 en tant qu'azureurs optiques.